# EUROPEAN PATENT SPECIFICATION

(11) **EP 2 914 216 B1**
(45) Date of publication and mention of the grant of the patent: **18.01.2017**
(21) Application number: 13782678.0
(22) Date of filing: 17.10.2013
(51) Int. Cl.: A61F 2/97, A61F 2/966, A61F 2/95

(54) **CATHETER DELIVERY SYSTEM TO RELEASE A SELF-EXPANDING IMPLANT**
KATHETERFREISETZUNGSSYSTEM ZUR FREISETZUNG EINES SELBSTEXPANDIERENDEN IMPLANTATS
SYSTÈME D'ADMINISTRATION À CATHÉTER POUR LA LIBÉRATION D'UN IMPLANT AUTO-DILATABLE

(30) Priority: 30.10.2012 NL 2009726
(43) Date of publication of application: 09.09.2015
(73) Proprietor: Angiomed GmbH & Co. Medizintechnik KG, 76227 Karlsruhe (DE)
(72) Inventor: DORN, Jürgen, 68809 Neulußheim (DE); HOFFMANN, Martina, 76297 Stutensee (DE); VOGEL, Michael, 76149 Karlsruhe (DE)
(74) Representative: Hoffmann Eitle
(86) International application number: PCT/EP2013/071713
(87) International publication number: WO 2014/067787

(56) References cited:
- EP-A1- 1 844 739
- WO-A1-2010/120671

## Description

### Technical Field

This invention relates to a percutaneous trans-luminal catheter delivery system for an implant, the system comprising a catheter shaft with a distal end to carry the implant and a proximal end, and an elongate housing at the proximal end that an operator can grasp when deploying the implant, the shaft having an inner push component which can push on the implant in a distal direction and an outer sheath component which radially surrounds the implant until said deployment of the implant into the bodily lumen, the outer sheath component being capable of being pulled proximally, from the housing, to deploy the implant, the housing being mounted on a proximal end portion of the push component and defining a channel, the housing containing a sheath slitter that is fixedly mounted relative to the length of the channel and that can slit the outer sheath as the outer sheath is caused to move proximally in the channel relative to the slitter.

### Background

Catheter delivery systems for trans-luminal delivery of implants, particularly self-expanding stents, have a rich history in the patent literature. Early proposals were for a simple sheath radially surrounding the radially-compressed stent at the distal end of the catheter system, the sheath being pulled back proximally, to release the stent from its bed, progressively, starting at its distal end of the bed, within the stenting site or stenosis of the bodily lumen in which the catheter delivery system had been advanced. Readers will appreciate that, because the stent is self-expanding, it is pressing on the luminal surface of the surrounding sheath, up to the moment of its release from the sheath. Thus, friction forces between the stent and the surrounding sheath must be taken into account when devising a delivery system that will allow the sheath to slide proximally over the full length of the outwardly-pushing, self-expanding stent.

The problems of friction will increase with the length of the stent, and the pressure on delivery system designers is to deliver ever-longer stents. Furthermore, there is steady pressure on stent delivery system designers to come up with systems that have ever-smaller passing diameters at the distal end of the catheter. The conventional unit of dimensions for diameters of systems to advance along a bodily lumen is the "French" which is one third of a millimeter. Thus, one millimeter is "3 French". To be able to reduce the passing diameter of a delivery system, for example from 7 French to 6 French, is a notable achievement.

One way to respond to the challenge of friction forces between a proximally withdrawing sheath and a self-expanding stent confined within it is to adopt a "rolling membrane" sheath system, in which the sheath is at least double the length of the stent that it surrounds, being doubled back on itself at a point distally beyond the distal end of the stent. Then, proximal withdrawal of the radially outer doubled back portion of the sheath length will cause the "rolling edge" between the outer and inner sheath portions to retreat proximally, rolling proximally down the length of the stent, to release the stent progressively, as with a single layer surrounding sheath.

Regardless of whether a conventional or rolling membrane sheath system is employed at the distal end of a stent delivery system, the delivery system requires some form of deployment mechanism provided at the proximal end of the stent delivery system to enable an operator to control at the proximal end the deployment of the distally located stent inside a patient. Typically, the stent is provided on the distal end of a push rod that extends from the proximal end to the distal end of the system. With this push rod held stationary, the user operates such a mechanism at the proximal end, resulting in the sheath system being pulled back, thereby deploying the stent, as described above.

One stent deployment mechanism is disclosed in US 2007/0244540 A1 (here "D1"). This mechanism involves the use of a thumb slider that is repeatedly translated distally and proximally, with each progressive proximal movement effecting progressive retraction of the sheath. A disadvantage of this deployment mechanism is the inability to deploy the stent in only one, or at least only a few, translations of the deployment mechanism. For lengthy stents, deploying the stent using this mechanism would prove a laborious task, requiring many translations. However, once the distal end of the implant is in place on the wall of the lumen in the body that is receiving the implant, a swift retraction of the sheath, to deploy the remaining length of the implant in one smooth stroke, is not available from this device.

D1 teaches the attractiveness of a hand unit that is physically small. The sheath of D1 is not a roll back membrane. Were it to be a roll back membrane, the distance it would have to be pulled back proximally would be doubled. The present invention aims to provide a simple and easy to manufacture hand unit that is small in size but yet is capable of deploying a lengthy implant covered by a roll back membrane.

US-A1-2010/0268243 Cook (here "D2") discloses a system to deploy a stent in which a tube is split with a cutting instrument as the tube is withdrawn proximally to deploy the stent. The split tube can be wound up on a spool or simply pulled out of the handle.

US-B2-7837725 Abbott (here "D3") also discloses a catheter device for delivering a stent in which an outer tubular member of the catheter is withdrawn proximally to release the stent, and the tubular member is split apart by blades, from its proximal end, whereby the length of the catheter can be reduced down to a length that is no longer than is necessary for the procedure for which it is destined to be used. For the deployment of the stent, a slider in a hand unit is used. Thus, an over-long hand unit would be needed, for deployment of a lengthy stent confined within a roll-back membrane. US-A-5687727 Danforth (here "D4") discloses a proximal adaptor for an over-the-wire angioplasty catheter. The adaptor facilitates exchange of catheters over a guidewire. It grips the guidewire and slits the catheter as the catheter is withdrawn proximally through the adaptor whereby the catheter can be removed completely by slitting it all the way to its distal end. Thereafter, a new catheter can be introduced and advanced along the *in situ* guidewire.

WO 2010/120671 A1 discloses a system that includes an outer catheter having an inner liner extending past the end of the outer catheter and an inner catheter disposed within the outer catheter. The inner liner is inverted and attached to the inner catheter. Relative movement between the outer and the inner catheters can urge the inner liner to peel away from the medical device. A handle is disposed at the proximal end of the outer catheter, and may include a splitter configured to slice the wall of the tubular member. The handle may also include a rotatable mechanism that can be attached to the tubular member. Rotation of the rotatable mechanism retracts a portion of the tubular member into the handle and winds the sliced portion about the rotatable mechanism.

The present invention aims to provide a system for deploying implants of any length, with a high degree of tactile feedback to the operator during deployment, and with optimal management of the forces of static and dynamic friction that occur during such deployment.

### Summary

According to the present invention, a catheter delivery system of the general form identified above is characterized in that the housing is slidable on the push component, the housing includes a clamp that can be actuated to clamp the housing to the push component to fix the housing lengthwise on the push component at any desired location along the push component and the housing defines an off-axis side channel for the slitted outer sheath proximal of the slitter, the side channel terminating at a pull aperture where the slitted sheath can be grasped and pulled whereby, with the housing clamped to the push element, the sheath can be pulled proximally, relative to the push component, thereby to deploy the implant.

With the present invention, the operator can hold the housing to keep the implant at the desired location in the body of the patient. The housing can be placed snug up against the percutaneous introducer through which the catheter enters the patient's body. Thus, the housing is separated from the distal end of the catheter by the minimum possible distance. The operator does not need to know in advance what this distance is. Simply, the catheter brings the implant to the implant site and then the operator brings the housing up to the introducer by advancing it distally along the shaft of the catheter, slitting the sheath as the housing advances.

With the housing so placed, the operator is well set to deploy the implant by pulling on the sheath. The line of tension from the operator's hand to the implant is straight except for any curvature in the bodily lumen in which the catheter shaft lies, and except for the angle in the housing that separates the housing axis and its side channel through which the slit sheath is pulled.

This is to be contrasted with the situation in devices in which the housing is not snug up against the introducer but, rather, separated from it by a bowed portion of the length of the catheter outside the body of the patient. Any such bowed portion increases the friction forces, defying the operator's efforts to pull back the sheath to deploy the implant. They can also be detrimental to the one-to-one relationship between a pull-back distance increment at the proximal end of the sheath and the resultant pull-back distance increment at the distal end of the sheath. In other words, with the invention, the operator is provided with as much tactile feedback as is possible, about how the implant deployment process is proceeding up at the distal end of the system.

Conveniently, the slitter is a single blade that makes a single longitudinal cut through the wall of the sheath. Nevertheless, it may be useful to make more than one cut in the sheath, notably, two cuts on opposite sides of the sheath, that is, one at each end of a diametral section through the axis of the sheath, to leave the slitted sheath in the form of two distinct ribbons.

The pull aperture of the housing is preferably closed by a cap. Prior to implant deployment, the system will need to be flushed of gas bubbles, using a flushing liquid (as is conventional). Of course, the housing will therefore require flushing components. In this regard, Luer connectors are ubiquitous. The cap might therefore incorporate a Luer connector element, and so might the proximal end of the axial channel. The cap can be used as a pulling knob if the proximal end of the slitted sheath is fixed to the cap that closes the pulling aperture.

The clamp which is needed to fix the housing to the push element where desired along the length of the push element can conveniently be provided at the proximal end of the channel, conveniently adjacent a flushing port at the proximal end of the channel. A rotatable collet is a convenient device for an operator to use, to lock the housing to the push component.

The housing preferably comprises a proximal portion and a distal strain relief portion, the distal strain relief portion being formed from a less rigid material and serving to reduce stress concentrations along the length of the catheter shaft distal of the more rigid proximal portion of the housing. This can prevent damage being induced in the catheter shaft during advancement into a patient and prior to deployment, which might otherwise result from undue bowing of the shaft.

In general, the architecture of the housing can be that of a so-called "Y-Adaptor", which is an element that, as such, is well-known to those skilled in the catheterization arts.

The invention aims to provide operators with a system with which deployment skills can readily be practised and improved. The system is intuitive to use, has a minimum of moving parts, and can be manufactured in simple steps (thereby enhancing the probability of unimpeached sterility).

If "quality" implies "predictability" then the systems of the invention can provide operators with an enhanced level of quality, at the same time being straightforward systems to graduate to, and to operate intuitively (and therefore reliably).

### Brief Description of the Drawings

For a better understanding of the present invention, and to show more clearly how the same may be carried into effect, reference will now be made, by way of example only, to the accompanying drawings, in which:
- Fig. 1: is the proximal end of a catheter delivery system in accordance with a first embodiment of the present invention with the catheter shaft extending through an introducer and terminating at the distal end as shown in either one of Figs. 2A or 2B;
- Fig. 2A: is the distal end of a catheter shaft carrying an implant and incorporating a pullback sheath;
- Fig. 2B: is the distal end of a catheter shaft carrying an implant and incorporating a rolling membrane;
- Fig. 3: is the proximal end of a catheter delivery system in accordance with a second embodiment of the present invention with the catheter shaft terminating at the distal end as shown in Fig. 4;
- Fig. 4: is the distal end of a catheter shaft carrying an implant and incorporating a rolling membrane; and
- Fig. 5: shows the sliding of the housing along the push component of the catheter shaft of a catheter delivery system in accordance with the present invention.

### Detailed Description

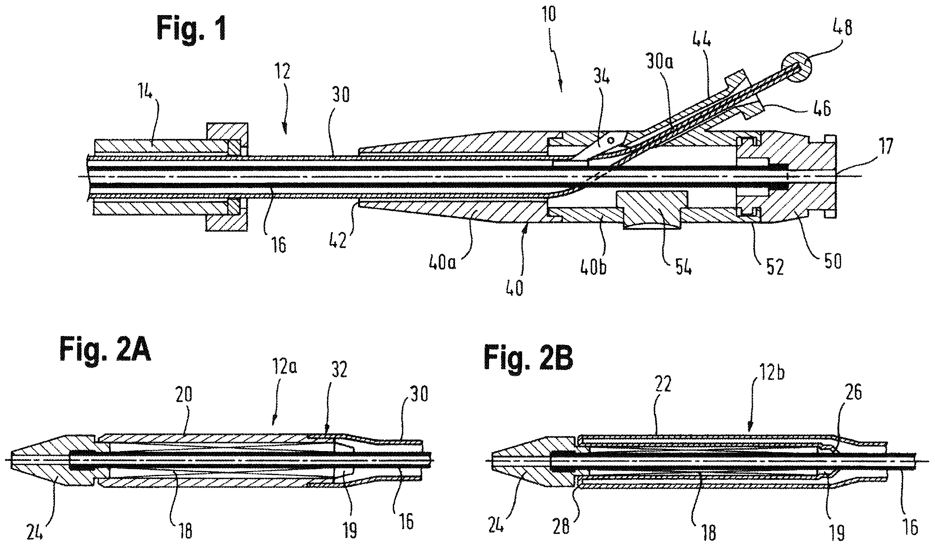

Fig. 1 shows the proximal end of a first embodiment of a catheter delivery system 10 with a catheter shaft 12 extending distally through an introducer 14 and terminating at the distal end in either one of the distal end catheter shaft configurations 12a or 12b, depicted in Figs. 2A and 2B respectively. The shaft 12 has an inner shaft 16 which is the push component of the catheter shaft and an outer sheath 30 which is the sheath component of the catheter shaft. The inner shaft 16 runs along a guidewire 17. As can be seen in Figs. 2A and 2B, a stent 18 is carried on the distal end of the inner shaft 16 and is positioned proximal of a tip 24 of the catheter and distal of a stent stop 19.

In Fig. 2A, the stent 18 is radially confined by a pullback sheath 20 that extends distally to the tip 24 of the catheter. In an overlapping zone 32 at the proximal end of the stent 18, the outer sheath 30 is bonded to the outside of the pullback sheath 20. Of course, the pullback sheath 20 and the outer sheath 30 could alternatively be formed as one.

In Fig. 2B, the stent 18 is radially confined this time by a rollback membrane 22 with one end 26 secured to the inner shaft 16 at a location just proximal of the stent stop 19, the membrane extending from this end 26 to the tip 24 of the catheter, at which point it reverses direction at a roll back annulus 28 and then advances proximally over the length of the stent 18. In this figure, no transition between the membrane 22 and the outer sheath 30 is shown, reflective of a unitary construction, but the membrane 22 and the outer sheath 30 could be formed separately and bonded, akin to the bonding between the pullback sheath 20 and the outer sheath 30 shown in Fig. 2A.

At the proximal end of the catheter shaft 12 there is provided an elongate housing 40 having a distal end 42 through which the catheter shaft 12 extends proximally. The housing 40 comprises two main portions, a distal strain relief portion 40a and a proximal grip portion 40b. The inner shaft 16 is slidably retained within a channel running through the length of the housing 40. The strain relief portion 40a is formed from a less rigid material than the grip portion 40b and, owing to its relative flexibility, serves to smooth out stress concentrations along the length of the catheter shaft 16 resulting from axial misalignment between the portion of the catheter shaft 16 in the section of channel associated with the relatively rigid grip portion 40b and the portion of the catheter shaft 16 distal of the channel of the housing 40.

The proximal end of the channel terminates in proximal end cap 50 formed from a female Luer connector element that receives the proximal end of the inner shaft 16 and is connected at the proximal end 52 of the housing 40.

At a fixed point along the channel of the housing 40, proximal of the distal end 42, a blade 34 is mounted to the housing 40. The blade 34 is longitudinally aligned to extend parallel with the axis of the catheter shaft 12, with the cutting surface facing distally, and extends through the outer surface of the outer sheath 30 so that a portion of the blade 34 protrudes radially inwards of the outer sheath 34. Although not apparent from Fig. 1, it is arranged that the radially innermost portion of the blade cannot bind on, or cut, the inner catheter.

Distal of the blade 34, the outer sheath 30 and inner shaft 16 are co-axial, with the outer sheath 30 surrounding the inner shaft 16. Proximal of the blade 34 however, outer sheath 30a, having been slitted by the blade 34, separates from the inner shaft 16, (which is unaffected by the blade 34) and extends proximally through the bore of an off-axis side channel 44, exiting through an aperture 46 and terminating in a pull knob 48. In the illustrated embodiment, the bore of the off-axis side channel 44 forms an acute angle of about 30° with the proximal direction of the channel of the housing 40. In that respect, the housing 40 resembles a well-known "Y-Adaptor".

In the embodiment shown in Fig. 1, the blade 34 is positioned at the same side on the circumference of the housing 40 as the off-axis side channel 44. This means that, when viewed along the longitudinal axis, the blade 40 is circumferentially offset from the off-axis side channel 44 if at all then by less than 90 degrees. The angular position of the blade 34 with respect to the bore of the off-axis side channel 44 will influence the cutting dynamics and ease of separation of the outer sheath 30 from the inner shaft 16.

On the outside surface of the housing 40 is provided a push button 54 that clamps down on the inner shaft 16 thereby preventing relative axial movement between the housing 40 and the inner shaft 16. In the illustrated embodiment, the portion of the push button 54 impacting upon the inner shaft 16 is provided with a high coefficient of friction to minimise slip. The push button 54 may be resiliently biased in the unclamped position to prevent unwanted clamping. There may be provided a latching mechanism to maintain the push button 54 in a clamped position upon being pushed radially inwards, which is released upon the push button 54 being pushed once again radially inwards.

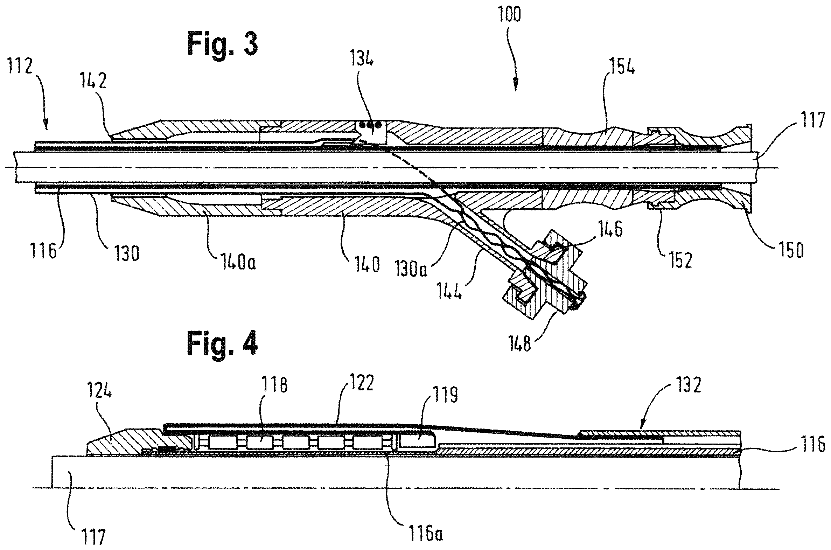

Fig. 3 shows the proximal end of a second embodiment of a catheter delivery system 100 with a catheter shaft 112 extending distally and terminating at a distal end as shown in Fig. 4. Only significant points of difference between this embodiment and the previous embodiment shall be discussed below.

The blade 134 is provided on the opposite side of the off-axis side channel 144. This means that, when viewed along the longitudinal axis, the blade 140 is circumferentially offset from the off-axis side channel 144 by more than 90 degrees. Preferably, the offset is 180°. In Fig. 4, this offset is 180 degrees. Positioning the blade 134 on the opposite side of the off-axis side channel 144 is understood to give a smoother cutting and divergence of the slitted outer sheath 130 from the inner catheter 116, as the slitted material of the outer sheath 130a can simply lift off the inner catheter 116 without there being required any degree of twisting of the outer sheath 130before it can enter the side channel 144.

The proximal end of the slitted portion of the outer sheath 130a proximal of the blade 134 terminates in a pull cap 148 formed from a Luer connector that is detachably connected to the aperture 146 of the off-axis side channel 144.

Rather than a push button 54, there is provided a collar, a rotatable collet 154 that may be rotated back and forth to move between a clamped position, in which there is exerted a clamping force on the inner catheter 116, and an unclamped position, in which the inner catheter 116 is free to slide within the channel of the housing 140.

At the distal end of the delivery system 100 shown in Fig. 4, there is this time provided an overlap section 132 in the rolling membrane 122, in which a portion of the membrane proximal of the overlap portion 132 is bonded to the outside of a portion distal of the overlap portion 132. It can also be seen that the inner catheter 116 is provided with a stent bed 116a on which the stent 118 is provided having a reduced circumference. This serves to reduce the lengthwise variation in the catheter thickness associated with the presence of the stent 118.

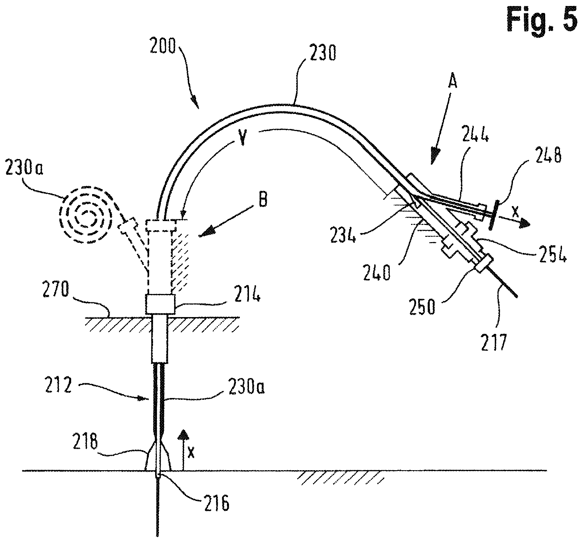

To illustrate the operation of the catheter delivery system, reference will be made to Fig. 5. The housing 240 of the delivery catheter system 200 is shown in two positions, an initial position A and a deployment position B.

In position A, e.g. following advancement of the catheter shaft 212 through an introducer 214 into a patient lumen to the desired stenting site in the lumen, there is a shaft length Y between the housing 240 and the introducer 214. The pull cap 248 and the proximal end cap 250 are released from the housing 240 and, ensuring that the clamping means 254 is not clamped to the inner shaft 216, whilst holding the proximal end cap 250 so as to prevent any movement of the distal end of the catheter shaft 212 in the patient lumen and optionally also holding the pull cap 248 so as to avoid bunching of the outer sheath 230 by maintaining tension in the vicinity of the cut, the housing 240 is slid distally along the length of the inner shaft 216 surrounded by the outer sheath 230. As the blade 234 in the housing 240 advances over the outer sheath 230, it forms a longitudinal cut in the outer sheath 230, and the point of separation between the inner shaft 216 and the outer sheath 230 advances with the blade 234. During this motion, the length of the slit outer sheath 230a that is proximal of the off-axis side channel 244 increases. Eventually, the housing 240 will meet with the introducer 214 and snugly fits close to, inside or in abutting contact therewith. The catheter delivery system 200 is then in position B, ready for deployment. At this point the clamping means 254 is activated to prevent relative movement between the housing 240 and the inner catheter 216, preventing unwanted movement of the stent 218.

With the housing 240 snugly against the introducer 214, either a section of the portion of the outer sheath 230a proximal of the off-axis side channel 244, or the pull cap 248, is pulled by a release distance X, thereby to deploy the stent 218.

At any point during the process, any adjustment of the position of the stent 218 relative to the patient lumen can be effected by pushing or pulling on the proximal end cap 250 with the clamping means 250 deactivated or by pushing or pulling on the housing 240 with the clamping means 250 activated.

## Claims

1. A percutaneous trans-luminal catheter delivery system (10) for an implant (118), the system comprising:
a catheter shaft (12) with a proximal end and a distal end to carry the implant, and
an elongate housing (40) at the proximal end that an operator can grasp when deploying the implant,
the shaft having an inner push component (16) which can push on the implant in a distal direction and an outer sheath component (22) which radially surrounds the implant until said deployment of the implant into the bodily lumen, the outer sheath component being capable of being pulled proximally, from the housing, to deploy the implant,
the housing being mounted on a proximal end portion of the push component and defining a channel,
the housing containing a sheath slitter (34) that is fixedly mounted relative to the length of the channel and that can slit the outer sheath as the outer sheath is caused to move proximally in the channel relative to the slitter,
the system being **characterized in that**:
the housing is slidable on the push component,
the housing includes a clamp (54) that can be actuated to clamp the housing to the push component to fix the housing lengthwise on the push component at any desired location along the push component, and
the housing defines an off-axis side channel (44) for the slitted outer sheath proximal of the slitter, the side channel terminating at a pull aperture (46) where the slitted sheath can be grasped and pulled whereby, with the housing clamped to the push element, the sheath can be pulled proximally, relative to the push component, thereby to deploy the implant.

2. System according to claim 1, wherein the slitter comprises a blade which makes a single cut through the sheath parallel to the axis of the sheath.

3. System as claimed in claim 1 or 2, wherein the pull aperture is closed by a cap (148) that is removable from the housing prior to deployment of the implant.

4. System as claimed in claim 3, wherein the proximal end of the slitted sheath is fixed to the cap so that the cap, when removed from the housing, can be grasped as a knob, and used to pull the slitted sheath proximally, to deploy the implant.

5. System as claimed in any one of the preceding claims, wherein the channel has a proximal end that ends in a female Luer connector element.

6. System as claimed in any one of the preceding claims, wherein the housing includes a clamp for clamping the housing to a cylindrical radially outside surface of the push component.

7. System as claimed in claim 6, wherein the clamp (154) can be actuated by a rotational movement of a collar on the housing, around the axis of the channel.

8. System as claimed in claim 6, wherein the clamp comprises a squeezable element that with finger pressure can be urged into frictional contact with the cylindrical surface.

9. System as claimed in claim 8, wherein the squeezable element is an elastomeric pad.

10. System as claimed in any one of claims 6 to 9, wherein the clamp is located at the proximal end of the housing.

11. System as claimed in any one of the preceding claims, wherein the side channel has a longitudinal axis that lies at an acute angle to the proximal direction of the channel in the housing.

12. System as claimed in claim 11, wherein the housing has the overall form of a "Y-Adaptor".

## Patentansprüche

1. Perkutanes transluminales Kathetereinführsystem (10) für ein Implantat (118), wobei das System umfasst:
einen Katheterschaft (12) mit einem proximalen Ende und einem distalen Ende zum Tragen des Implantats, und
ein längliches Gehäuse (40) am proximalen Ende, welches ein Benutzer beim Einsetzen des Implantats greifen kann,
wobei der Schaft ein inneres Schiebebauteil (16) aufweist, welches in einer distalen Richtung auf das Implantat drücken kann, und ein äußeres Ummantelungsbauteil (22) aufweist, welches das Implantat bis zum Einsetzen des Implantats in das Körperlumen radial umgibt, wobei das äußere Ummantelungsbauteil geeignet ist, proximal, vom Gehäuse, gezogen zu werden, um das Implantat einzusetzen,
wobei das Gehäuse auf einem proximalen Endabschnitt des Schiebebauteils angebracht ist und einen Kanal definiert,
wobei das Gehäuse ein Schlitzmesser (34) für die Ummantelung enthält, das relativ zur Länge des Kanals fest angebracht ist und die äußere Ummantelung aufschlitzen kann, wenn die äußere Ummantelung veranlasst wird, sich relativ zum Schlitzmesser proximal in den Kanal zu bewegen,
wobei das System **dadurch gekennzeichnet ist, dass**:
das Gehäuse auf dem Schiebebauteil verschiebbar ist,
das Gehäuse eine Klemme (54) umfasst, die betätigt werden kann, um das Gehäuse am Schiebebauteil festzuklemmen und so das Gehäuse der Länge nach auf dem Schiebebauteil an jeder gewünschten Stelle entlang des Schiebebauteils zu fixieren, und
das Gehäuse einen achsversetzten Seitenkanal (44) für die aufgeschlitzte äußere Ummantelung proximal zum Schlitzmesser definiert, wobei der Seitenkanal an einer Zugöffnung (46) endet, wo die aufgeschlitzte Ummantelung gegriffen und gezogen werden kann, wobei die Ummantelung bei am Schiebebauteil angeklemmtem Gehäuse relativ zum Schiebebauteil proximal gezogen werden kann, um dadurch das Implantat einzusetzen.

2. System nach Anspruch 1, wobei das Schlitzmesser eine Klinge umfasst, welche einen einzelnen Schnitt durch die Ummantelung parallel zur Achse der Ummantelung macht.

3. System wie in Anspruch 1 oder 2 beansprucht, wobei die Zugöffnung von einer Kappe (148) verschlossen ist, die vor dem Einsetzen des Implantats vom Gehäuse entfernbar ist.

4. System wie in Anspruch 3 beansprucht, wobei das proximale Ende der aufgeschlitzten Ummantelung an der Kappe fixiert ist, so dass die Kappe beim Entfernen vom Gehäuse wie ein Knauf gegriffen werden kann und verwendet werden kann, um die aufgeschlitzte Ummantelung proximal zu ziehen und so das Implantat einzusetzen.

5. System wie in einem der vorangehenden Ansprüche beansprucht, wobei der Kanal ein proximales Ende aufweist, das in einem weiblichen Luer-Verbinderelement endet.

6. System wie in einem der vorangehenden Ansprüche beansprucht, wobei das Gehäuse eine Klemme zum Anklemmen des Gehäuses an einer zylindrischen radial außenseitigen Oberfläche des Schiebebauteils umfasst.

7. System wie in Anspruch 6 beansprucht, wobei die Klemme (154) durch eine Drehbewegung eines Kragens auf dem Gehäuse um die Achse des Kanals betätigt werden kann.

8. System wie in Anspruch 6 beansprucht, wobei die Klemme ein zusammendrückbares Element umfasst, das mit Fingerdruck in eine Reibberührung mit der zylindrischen Oberfläche gedrängt werden kann.

9. System wie in Anspruch 8 beansprucht, wobei das zusammendrückbare Element ein elastomeres Polster ist.

10. System wie in einem der Ansprüche 6 bis 9 beansprucht, wobei sich die Klemme am proximalen Ende des Gehäuses befindet.

11. System wie in einem der vorangehenden Ansprüche beansprucht, wobei der Seitenkanal eine Längsachse aufweist, die unter einem spitzen Winkel zur proximalen Richtung des Kanals im Gehäuse liegt.

12. System wie in Anspruch 11 beansprucht, wobei das Gehäuse insgesamt die Form eines "Y-Adapters" aufweist.

## Revendications

1. Système de distribution de cathéter transluminal percutané (10) pour un implant (118), le système comprenant :
une tige de cathéter (12) comprenant une extrémité proximale et une extrémité distale pour transporter l'implant; et
un logement allongé (40) à l'extrémité proximale que peut saisir un opérateur lors du déploiement de l'implant ;
la tige possédant un composant de poussée interne (16) qui peut exercer une poussée sur l'implant dans une direction distale et un composant de gaine externe (22) qui entoure l'implant dans une direction radiale jusqu'audit déploiement de l'implant dans la lumière corporelle, le composant de gaine externe pouvant être tiré en direction proximale, par rapport au logement, afin de déployer l'implant;
le logement étant monté sur une portion terminale proximale du composant de poussée et définissant un canal ;
le logement contenant un dispositif de coupe de gaine (34) qui est monté à demeure par rapport à la longueur du canal et qui peut fendre la gaine externe lorsque la gaine est amenée à se déplacer en direction proximale dans le canal par rapport au dispositif de coupe ;
le système étant **caractérisé en ce que** :
le logement peut glisser sur le composant de poussée ;
le logement englobe une pince (54) qui peut être activée pour serrer le logement contre le composant de poussée afin de fixer le logement en longueur sur le composant de poussée à n'importe quel endroit désiré le long du composant de poussée ; et
le logement définit un canal latéral désaxé (44) pour la gaine externe fendue, en position proximale par rapport au dispositif de coupe, le canal latéral se terminant à un orifice de traction (46) où la gaine fendue peut être saisie et tirée, d'une manière telle que lorsque le logement est serré contre l'élément de poussée, la gaine peut être tirée en direction proximale, par rapport au composant de poussée, pour ainsi déployer l'implant.

2. Système selon la revendication 1, dans lequel le dispositif de coupe comprend une lame qui pratique une seule coupure à travers la gaine parallèlement à l'axe de la gaine.

3. Système selon la revendication 1 ou 2, dans lequel l'orifice de traction est fermé par un capuchon (148) qui peut être retiré du logement avant le déploiement de l'implant.

4. Système selon la revendication 3, dans lequel l'extrémité proximale de la gaine fendue est fixée au capuchon d'une manière telle que le capuchon, lorsqu'il est retiré du logement, peut être saisi à la manière d'un bouton et peut être utilisé pour tirer la gaine fendue en direction proximale, afin de déployer l'implant.

5. Système selon l'une quelconque des revendications précédentes, dans lequel le canal possède une extrémité proximale qui se termine par un élément sous la forme d'un raccord Luer femelle.

6. Système selon l'une quelconque des revendications précédentes, dans lequel le logement comprend une pince pour serrer le logement contre une surface radialement externe cylindrique du composant de poussée.

7. Système selon la revendication 6, dans lequel la pince (154) peut être activée via un mouvement rotatif d'une bague sur le logement, autour de l'axe du canal.

8. Système selon la revendication 6, dans lequel la pince comprend un élément compressible qui, avec la pression des doigts, peut être poussé en contact à friction avec la surface cylindrique.

9. Système selon la revendication 8, dans lequel l'élément compressible est un tampon élastomère.

10. Système selon l'une quelconque des revendications 6 à 9, dans lequel la pince est disposée à l'extrémité proximale du logement.

11. Système selon l'une quelconque des revendications précédentes, dans lequel le canal latéral possède un axe longitudinal qui forme un angle aigu par rapport à la direction proximale du canal dans le logement.

12. Système selon la revendication 11, dans lequel le logement possède la forme globale d'un « adaptateur en Y ».
